Europäisches Patentamt

European Patent Office

Office européen des brevets

(19)

(11) **EP 1 514 513 A1**

(12) **EUROPEAN PATENT APPLICATION**

(43) Date of publication:
**16.03.2005 Bulletin 2005/11**

(51) Int Cl.⁷: **A61B 5/053**

(21) Application number: **04020164.2**

(22) Date of filing: **25.08.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **12.09.2003 JP 2003320557**

(71) Applicant: **TANITA CORPORATION Tokyo (JP)**

(72) Inventor: **Itagaki, Shuji Tokyo (JP)**

(74) Representative: **Grünecker, Kinkeldey, Stockmair & Schwanhäusser Anwaltssozietät Maximilianstrasse 58 80538 München (DE)**

(54) **Body composition data acquiring apparatus**

(57)     A body composition data acquiring apparatus has a main unit comprising: bioelectrical impedance measuring means, body weight measuring means, leg length measuring means, and body composition data calculating means wherein the bioelectrical impedance measuring means measures a bioelectrical impedance between both feet by use of a group of electrodes which make contact with the bottoms of the feet of a subject, the body weight measuring means measures the body weight of the subject, the leg length measuring means measures the leg length of the subject, and the body composition data calculating means calculates the body composition data of the subject based on at least the bioelectrical impedance, body weight and leg length measured by these measuring means.

FIG. 1

**Description**

BACKGROUND OF THE INVENTION

(i) Field of the Invention

This invention relates to a body composition data acquiring apparatus which acquires body composition data such as a body fat mass (percentage), a total body water amount (percentage), a muscle mass (percentage), a visceral fat area (amount), a bone mass, BCM (Body Cell Mass) and a basal metabolic rate by measuring the bioelectrical impedance of a subject. More specifically, it relates to a body composition data acquiring apparatus which acquires body composition data from at least a bioelectrical impedance between both feet of a subject and the body weight and leg length of the subject.

(ii) Description of the Related Art

Body composition data acquiring apparatuses which acquire body composition data such as a body fat mass (percentage), a total body water amount (percentage), a muscle mass (percentage), a visceral fat area (amount), a bone mass, BCM (Body Cell Mass) and a basal metabolic rate by measuring the bioelectrical impedance of a subject have been currently becoming widely used in a number of general households. A so-called scale-type body fat monitor which is particularly well-known as a type of the body composition data acquiring apparatuses has a group of electrodes for measuring a bioelectrical impedance on the top surface of the scale and calculates body composition data such as a body fat mass (percentage) from a bioelectrical impedance between both feet which is measured by use of the electrode group and data of a subject such as a body height, a body weight, gender and age, by use of appropriate regression computing equations according to a known bioelectrical impedance analysis (for example, refer to Patent Publication 1).

Further, another scale-type body fat monitor which is a combination of the above scale-type body fat monitor and an electronic body height meter is also known, and the fat monitor is capable of automatically measuring a body height as well as a body weight and a bioelectrical impedance (for example, refer to Patent Publications 2 and 3) . In addition, a body fat monitor which uses the path length between body parts where a bioelectrical impedance is measured as data about the length of an electric conductor is proposed (for example, refer to Patent Publication 4).

    Patent Publication 1
        Japanese Patent [Publication No. 5-49050]
    Patent Publication 2
        Japanese Patent No. 2,835,662
    Patent Publication 3

        Japanese Patent Publication No. 2000-41966
    Patent Publication 4
        Japanese Patent Publication No. 2001-29321

A scale-type body fat monitor as disclosed in Patent Publication 1 measures a body weight and a bioelectrical impedance automatically but requires a subject to manually enter data such as a body height, gender and age. The body height in particular is an essential input item since acquirement of body composition data in accordance with the bioelectrical impedance analysis is based on the length, area and resistivity of an electric conductor. However, the body height cannot be input in the same manner as gender is input, i.e., by selecting one of two choices, so that a subject must know his own body height and generally input a three-digit number. Elderly people in particular often feel that the input of a three-digit number is complicated. Further, they may already have difficulty in remembering their own body heights, or even if they do remember their own body heights, the body heights may already be inaccurate because people shrink slightly in height as they get older. Consequently, an inaccurate body height may be input and inaccurate body composition data may therefore be acquired, with the result that use of a body composition data acquiring apparatus such as the scale-type body fat monitor is liable to be avoided.

Meanwhile, a scale-type body fat monitor as disclosed in Patent Publications 2 and 3 is capable of automatically measuring a body height as well as a body weight and a bioelectrical impedance. However, the scale-type body fat monitor incorporating an electronic body height meter is large in size and is not suitable for use in general households although it may be suitable for use in medical institutions.

Further, it is questioned whether it is appropriate in the first place to use a body height as data about the length of an electric conductor in a body composition data acquiring apparatus using a bioelectrical impedance analysis. That is, for example, since not a few elderly people have a bent back, a body height measured by an electronic body height meter is not necessarily appropriate data as data about the length of an electric conductor used in the bioelectrical impedance analysis. Particularly, in the case of a body composition data acquiring apparatus which measures a bioelectrical impedance by passing a current between both feet such as the above scale-type body fat monitor, it is considered desirable to use the length of the leg which is an actual current path as data about the length of an electric conductor.

In this regard, the body fat monitor disclosed in Patent Publication 4 takes the length from the crotch to the bottoms of the feet of a subject as the path length between body parts to be measured when acquiring a body fat mass by measuring a bioelectrical impedance between both feet, for example. However, in the case of the body fat monitor of Patent Publication 4, although it is proposed to provide the fat monitor with a measuring tape so as to measure the length from the crotch to the bottoms of the feet, a subject must carry out the measurement of the length from the crotch to the bottoms of the feet separately and enter the measurement value by means of keys, and it is difficult to say that the apparatus is easy to use for elderly people in particular.

Thus, an object of the present invention is to solve the above problems and provide a body composition data acquiring apparatus which can measure data appropriate as data about the length of an electric conductor in a bioelectrical impedance analysis automatically without causing a subject to perform complicated input operations and use the measured data for acquiring body composition data.

SUMMARY OF THE INVENTION

A body composition data acquiring apparatus of the present invention is a body composition data acquiring apparatus having a main unit comprising:

    bioelectrical impedance measuring means,
    body weight measuring means,
    leg length measuring means, and
    body composition data calculating means

wherein

    the bioelectrical impedance measuring means measures a bioelectrical impedance between both feet by use of a group of electrodes which make contact with the bottoms of the feet of a subject,
    the body weight measuring means measures the body weight of the subject,
    the leg length measuring means measures the leg length of the subject, and
    the body composition data calculating means calculates the body composition data of the subject based on at least the bioelectrical impedance, body weight and leg length measured by these measuring means.

The leg length measuring means may measure the length from the crotch to the bottoms of the feet of the subject.

Alternatively, the leg length measuring means may measure the length from the knees to the bottoms of the feet of the subject.

Further, the leg length measuring means may comprise: a pole which is positioned nearly perpendicularly to the main unit,

    a cursor which is slidably attached to the pole, and
    a position sensor which detects the position of the cursor.

Alternatively, the leg length measuring means may comprise:

    an ultrasonic wave transmitter which is provided on the top surface of the main unit and transmits an ultrasonic wave upward, and
    an ultrasonic wave receiver which is provided on the top surface of the main unit and receives an ultrasonic wave from above.

Alternatively, the leg length measuring means may comprise:

    a light emitter which is provided on the top surface of the main unit and emits light upward, and
    a light receiver which is provided on the top surface of the main unit and receives light from above.

Alternatively, the leg length measuring means may comprise:

    a flexible member which can be pulled out of the main unit and
    has a grip at one end, and
    a pulled length sensor which detects the pulled length of the flexible member.

Further, the grip of the flexible member may have a group of electrodes for measuring a bioelectrical impedance.

In the body composition data acquiring apparatus of the present invention, the length of the leg which is a current path in measuring a bioelectrical impedance between both feet is measured automatically by the leg length measuring means incorporated in the main unit. Thereby, the length of the leg which is conceived more appropriate than a body height as data about the length of an electric conductor which is used in a bioelectrical impedance analysis is used in calculations of body composition data, so that more accurate estimations of body composition data than estimations using a body height can be expected and a subject does not need to perform complicated operations such as remembering his/her body height or length form the crotch to the bottoms of the feet or measuring the data separately and inputting the data.

In particular, when the length from the crotch to the bottoms of the feet of a subject is measured by the leg length measuring means, the length which is nearly equivalent to an actual current path is used in calculations of body composition data, so that further more accurate estimations of body composition data can be expected.

Further, it is known that the length from the knees to the bottoms of the feet undergoes a very small change caused by aging. Therefore, when the length from the knees to the bottoms of the feet of a subject is measured by the leg length measuring means, there is no need to concern about a change in the length (length from the knees to the bottoms of the feet) caused by aging, so that once the length is measured, the measured length can be used over a long time period.

Further, when the leg length measuring means comprises a pole which is positioned nearly perpendicularly to the main unit, a cursor which is slidably attached to the pole, and a position sensor which detects the position of the cursor, a subject can measure the length from the crotch to the bottoms of the feet or the length from the knees to the bottoms of the feet of the subject automatically by moving the cursor up or down to the height of the crotch or knees of the subject.

Further, when the leg length measuring means comprises an ultrasonic wave transmitter which is provided on the top surface of the main unit and transmits an ultrasonic wave upward and an ultrasonic wave receiver which is provided on the top surface of the main unit and receives an ultrasonic wave from above, an ultrasonic wave is transmitted from the ultrasonic wave transmitter with a subject standing on the top surface of the main unit, and the ultrasonic wave reflected at the crotch of the subject (reflected wave) is received by the ultrasonic wave receiver. Consequently, the length from the crotch to the bottoms of the feet of the subject is measured automatically from a time taken from transmission of the wave to receipt of the wave.

Further, when the leg length measuring means comprises a light emitter which is provided on the top surface of the main unit and emits light upward and a light receiver which is provided on the top surface of the main unit and receives light from above, light is emitted from the light emitter with a subject standing on the top surface of the main unit, and the light reflected at the crotch of the subject (reflected light) is received by the light receiver. Consequently, the length from the crotch to the bottoms of the feet of the subject is measured automatically from the distance between the light emitter and the light receiver, the light emitting angle in the light emitter and the light receiving angle in the light receiver.

Further, when the leg length measuring means comprises a flexible member which can be pulled out of the main unit and has a grip at one end and a pulled length sensor which detects the pulled length of the flexible member, a subject holds the grips, pulls out the flexible members and stands on the top surface of the main unit with the hands on both sides of the waist, whereby the length which is nearly equivalent to the length from the crotch to the bottoms of the feet of the subject is measured automatically. When electrodes for measuring a bioelectrical impedance are provided on the grips, a bioelectrical impedance between the hand and the foot can also be measured by the above electrodes in combination with the electrodes which make contact with the bottoms of the feet.

BRIEF DESCRIPTION OF THE DRAWINGS

Fig. 1 is an external perspective view of a body composition data acquiring apparatus as a first embodiment of the present invention.

Fig. 2 is a block diagram showing the constitution of an electric circuit in the body composition data acquiring apparatus of the present invention.

Fig. 3 is a flowchart showing the flow of control processes executed in the body composition data acquiring apparatus of the present invention.

Fig. 4 is a diagram showing examples of screen images displayed on the display unit of the body composition data acquiring apparatus of the present invention.

Fig. 5 is an external perspective view of a body composition data acquiring apparatus as a second embodiment of the present invention.

Fig. 6 is an external perspective view of a body composition data acquiring apparatus as a third embodiment of the present invention.

Fig. 7 is a diagram for explaining a principle of a light receiver.

Fig. 8 is an external perspective view of a body composition data acquiring apparatus as a fourth embodiment of the present invention.

Fig. 9 is a diagram for explaining a principle of a pulled length sensor.

DETAILED DESCRIPTION OF THE PREFERRED EMBODIMENTS

A body composition data acquiring apparatus of the present invention is a body composition data acquiring apparatus having a main unit comprising:

bioelectrical impedance measuring means,
body weight measuring means,
leg length measuring means, and
body composition data calculating means

wherein

the bioelectrical impedance measuring means measures a bioelectrical impedance between both feet by use of a group of electrodes which make contact with the bottoms of the feet of a subject,
the body weight measuring means measures the body weight of the subject,
the leg length measuring means measures the leg length of the subject, and
the body composition data calculating means calculates the body composition data of the subject based on at least the
bioelectrical impedance, body weight and leg length measured by these measuring means.

The leg length measuring means may measure the length from the crotch to the bottoms of the feet of the subject.

Alternatively, the leg length measuring means may measure the length from the knees to the bottoms of the feet of the subject.

Further, the leg length measuring means may comprise:

a pole which is positioned nearly perpendicular to the main unit,
a cursor which is slidably attached to the pole, and
a position sensor which detects the position of the cursor.

Alternatively, the leg length measuring means may comprise:

an ultrasonic wave transmitter which is provided on the top surface of the main unit and transmits an ultrasonic wave upward, and
an ultrasonic wave receiver which is provided on the top surface of the main unit and receives an ultrasonic wave from above.

Alternatively, the leg length measuring means may comprise:

a light emitter which is provided on the top surface of the main unit and emits light upward, and
a light receiver which is provided on the top surface of the main unit and receives light from above.

Alternatively, the leg length measuring means may comprise:

a flexible member which can be pulled out of the main unit and
has a grip at one end, and
a pulled length sensor which detects the pulled length of the flexible member.

Further, the grip of the flexible member may have a group of electrodes for measuring a bioelectrical impedance.

Embodiment 1

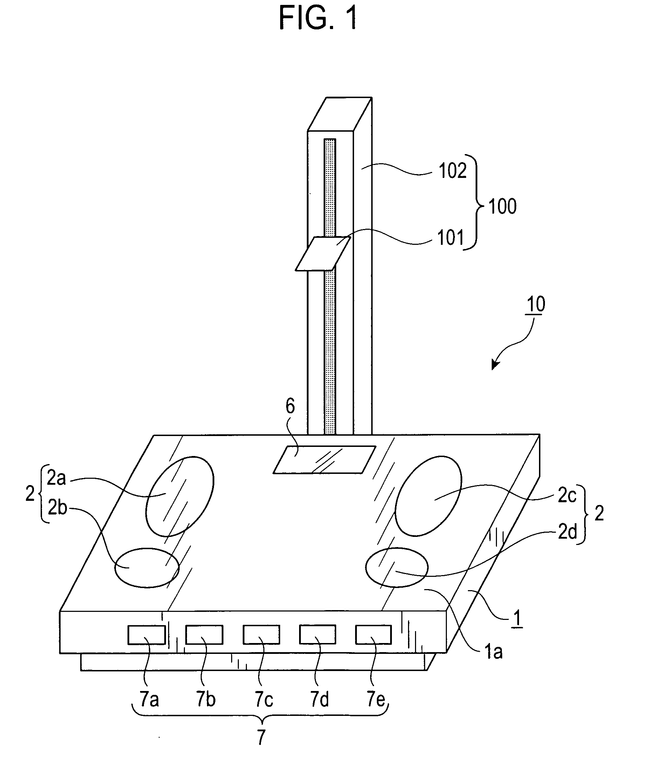

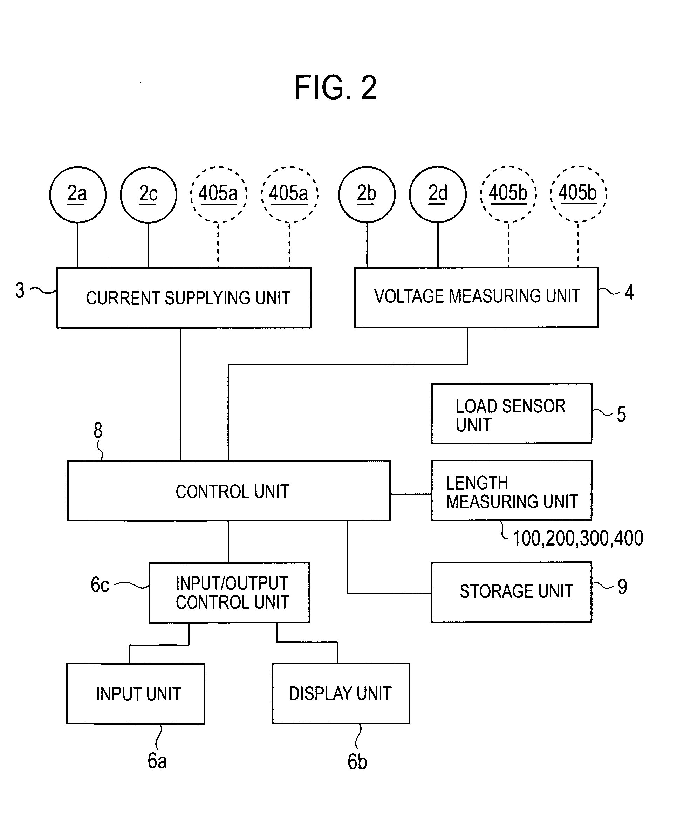

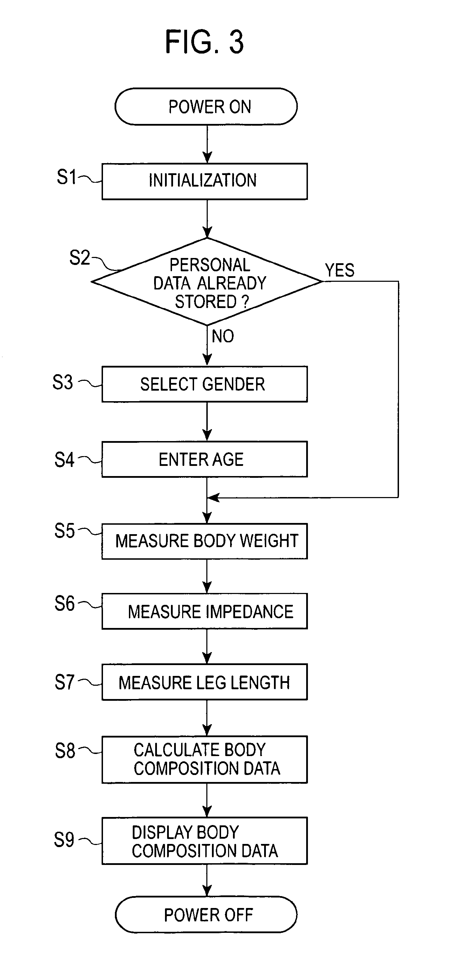

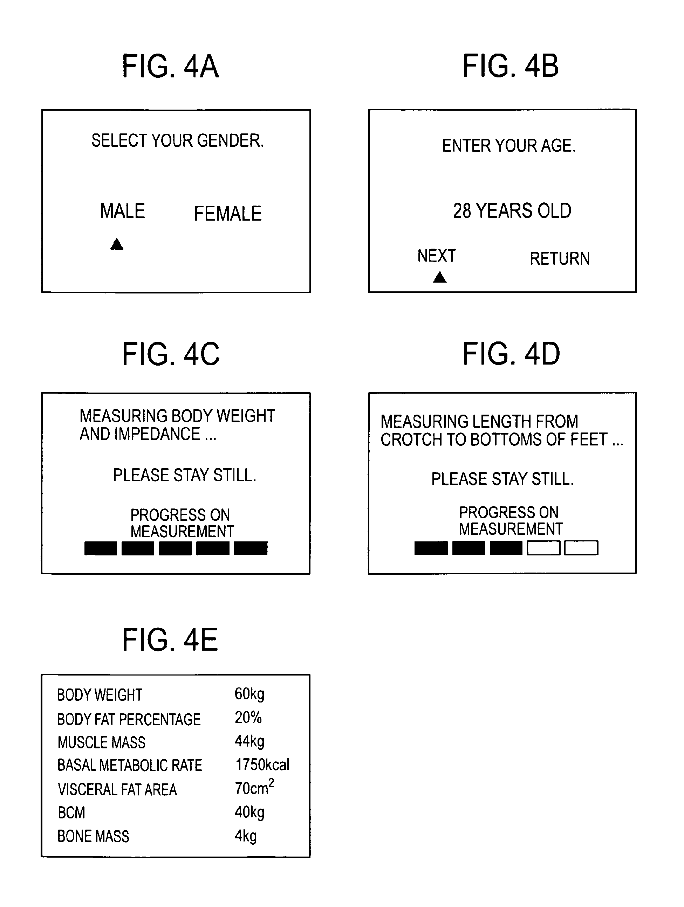

Fig. 1 is an external perspective view of a body composition data acquiring apparatus 10 as a first embodiment of the present invention, and Fig. 2 is a block diagram showing an overview of an electric circuit incorporated in the body composition data acquiring apparatus 10. Fig. 3 is a flowchart showing the flow of control processes in acquiring body composition data by use of the body composition data acquiring apparatus 10, and Fig. 4 is a diagram showing examples of screen images displayed on the display unit of the body composition data acquiring apparatus 10.

The body composition data acquiring apparatus 10 has a main unit 1. On the top surface 1a of the main unit 1, an electrode group 2 comprising electrodes 2a and 2b which make contact with the bottom of the left foot of a subject and electrodes 2c and 2d which make contact with the bottom of the right foot of the subject. The electrodes 2a and 2c are connected to a current supplying unit 3 which is incorporated in the main unit 1 and make contact with the toe sides of the bottoms of both feet so as to supply a weak alternating current to between both feet. Meanwhile, the electrodes 2b and 2d are connected to a voltage measuring unit 4 which is incorporated in the main unit 1 and make contact with the heel sides of the bottoms of both feet so as to measure a potential difference between both feet. The current supplying unit 3 and the voltage measuring unit 4 are connected to a control unit 8 where a bioelectrical impedance between both feet is measured (calculated) from the current value supplied to between both feet and the potential difference (voltage value) measured between both feet based on the Ohm's law. That is, these electrode group 2, current supplying unit 3, voltage measuring unit 4 and control unit 8 primarily constitute bioelectrical impedance measuring means. In the electrode group 2, the electrodes 2a and 2c which make contact with the toe side may be connected to the voltage measuring unit 4 and used for measuring a potential difference, and the electrodes 2b and 2d which make contact with the heel side may be connected to the current supplying unit 3 and used for supplying a current.

Further, in the main unit 1, a load sensor unit 5 is incorporated in connection with the control unit 8. The load sensor unit 5 outputs an electrical signal corresponding to a load applied on the top surface 1a. When a subject stands on the top surface 1a, an electrical signal is output from the load sensor unit 5, and the control unit 8 measures (calculates) the body weight of the subject based on the electrical signal. That is, these load sensor unit 5 and control unit 8 primarily constitute body weight measuring means. In the present embodiment, the load sensor unit 5 is constituted by four load cells each comprising a strain gauge, and they are installed nearly on the four corners of the undersurface side of the main unit 1.

Further, in the main unit 1, an input/output unit 6 is incorporated. The input/output unit 6 comprises an input unit 6a which is used by a subject to enter personal data such as gender and age, a display unit 6b which displays, e.g., body composition data acquired by the body composition data acquiring apparatus 10, and an input/output control unit 6c which connects these input unit 6a and display unit 6b to the control unit 8. The input unit 6a may be constituted by key switches and the like, and the display unit 6b may be constituted by a liquid crystal screen or the like.

Further, in the main unit 1, a foot switch group 7 is provided. The foot switch group 7 comprises a power switch 7a and personal data switches 7b, 7c, 7d and 7e. At the press of the power switch 7a, electric power is supplied from a power unit (not shown) such as dry batteries installed in the main unit 1 to each unit, and at the re-press of the power switch 7a, the supply of the electric power is stopped. Further, the personal data switches 7b, 7c, 7d and 7e are used to retrieve personal data stored in a storage unit 9 which is incorporated in the main unit 1, and they will be further described later. It is also possible to enable each of these personal data switches 7b, 7c, 7d and 7e to work as a power switch.

Further, in the main unit 1, a length measuring unit 100 as leg length measuring means which is a characteristic constitution of the present invention is incorporated. The length measuring unit 100 comprises a pole 101 which is positioned nearly perpendicularly to the top surface 1a, a cursor 102 which is slidably attached to the pole 101, and a position sensor (not shown) which detects the position of the cursor 102. When the cursor 102 is moved upward or downward along the pole 101, an electrical signal corresponding to the distance from the cursor 102 to the top surface 1a of the main unit is sent from the position sensor to the control unit 8. In the control unit 8, a height indicated by the position of the cursor 102 is measured (calculated) based on the electrical signal. The position sensor may be constituted by, for example, a gear which rotates along with the upward or downward movement of the cursor 102 and an encoder which counts the number of rotations of the gear. Further, to ensure the reliability of body weight measurement by the load sensor unit 5, the pole 101 is more preferably fixed to the main unit base which contacts with the floor than to the main unit platform on which the electrode group 2 is provided.

Therefore, when a subject tries to measure the length of the leg, the subject stands upright on the top surface 1a and then adjusts the cursor 102 to the position corresponding to his/her crotch when he/she intends to measure the length from the crotch to the bottoms of the feet or adjusts the cursor 102 to the position corresponding to his/her knees when he/she intends to measure the length from the knees to the bottoms of the feet, thereby measuring the length from the crotch to the bottoms of the feet or the length from the knees to the bottoms of the feet as the leg length. That is, the length measuring unit 100 which comprises these pole 101, cursor 102 and position sensor and the control unit 8 primarily constitute leg length measuring means.

The height (length) of the pole 101 itself is satisfactorily about 80 cm for measurement of the length from the crotch to the bottoms of the feet and about 50 cm for measurement of the length from the knees to the bottoms of the feet, thereby making the whole system much smaller than an electronic body height meter or the like. Further, it is also possible to render the pole 101 detachable from or foldable toward the main unit 1 so as to improve ease of storage of the body composition data acquiring apparatus 10.

The operation of the body composition data acquiring apparatus 10 when a subject acquires body composition data by use of the apparatus 10 will be described with reference to the control flowchart in Fig. 3 and examples of screen images in Fig. 4.

When a subject presses down the power switch 7a, electric power is supplied to the electric circuit of the main unit 1 and the control unit 8 executes an initialization process in STEP S1. Then, when the subject presses any of the personal data switches 7b, 7c, 7d and 7e, it is determined in STEP S2 whether personal data corresponding to the pressed personal data switch is already stored in the storage unit 9. When the personal data is stored (Yes), the control unit 8 reads in the personal data and proceeds to STEP S5. When it is not stored (No), the control unit 8 proceeds to STEP S3.

In STEP S3, types of gender and a message urging the subject to select and enter either of the gender types are displayed on the display unit 6b as shown in Fig. 4A. When the subject selects and enters gender by use of the input unit 6a, the control unit 8 proceeds to STEP S4. In STEP S4, a message urging the subject to enter age is displayed on the display unit 6b as shown in Fig. 4B. When the subject enters age by use of the input unit 6a, the control unit 8 proceeds to STEP S5. These gender and age entered in STEPS S3 and S4 are stored in the storage unit 9 as personal data corresponding to the pressed personal data switch.

Then, in STEP S5, the body weight of the subject is measured, and in subsequent STEP S6, a bioelectrical impedance is measured. To be more specific, on the display unit 6b, a message urging the subject to stand on the top surface 1a of the main unit 1 with the bottoms of both feet of the subject in contact with the electrode group 2 and stay still for a given time (e.g., 10 seconds) is displayed. When the subject stands on the unit 1 by following the message, the body weight is measured by the load sensor unit 5 and the control unit 8 and the bioelectrical impedance is measured by the electrode group 2, the current supplying unit 3, the voltage measuring unit 4 and the control unit 8, as described above. During the measurements, such a screen image as shown in Fig. 4C is displayed on the display unit 6b.

Then, in STEP S7, the length from the crotch to the bottoms of the feet is measured as the leg length of the subject. To be more specific, on the display unit 6b, a message urging the subject to adjust the cursor 102 to the position corresponding to the crotch of the subject and stay still for a few seconds (e.g., 5 seconds) is displayed. When the subject follows the message, the length from the crotch to the bottoms of the feet is measured by the length measuring unit 100 and the control unit 8 as described above. During the measurement, such a screen image as shown in Fig. 4D is displayed on the display unit 6b. The measured length from the crotch to the bottoms of the feet may be stored in the storage unit 9 as personal data, in addition to the above gender and age.

Then, in STEP S8, in the control unit 8 as body composition data calculating means, the body composition data of the subj ect is calculated by substituting the gender and age entered in STEPS S3 and S4 and the body weight, bioelectrical impedance and length from the crotch to the bottoms of the feet which have been measured in STEPS S5 to S7 into predetermined regression computing equations, in which the gender, age, body weight, bioelectrical impedance and length from the crotch to the bottoms of the feet are preset as variables, and each of the predetermined regression computing equations are prepared in advance for each of the body composition data to be acquired. A variety of body composition data including a body fat mass, a body fat percentage, a visceral fat area, a visceral fat mass, a total body water amount, a total body water percentage, a muscle mass, a muscle percentage, a bone mass, BCM (Body Cell Mass) and a basal metabolic rate are expected as the body composition data to be calculated, and all of these data may be calculated or some specific data may be selected and calculated. Further, the above gender and age are desirably included in the regression computing equations so as to calculate the body composition data as highly accurately as possible, although they may be omitted from the calculations. In addition to or in place of the gender and age, other data such as race (yellow, white, black) may be added as variables.

Then, in STEP S9, the body composition data calculated in STEP S8 are displayed on the display unit 6b as shown in Fig. 4E. Upon passage of a given time from the press of the power switch 7a (i.e., from activation of the system), the supply of electric power from the power source is stopped automatically, thereby completing all control processes.

Embodiment 2

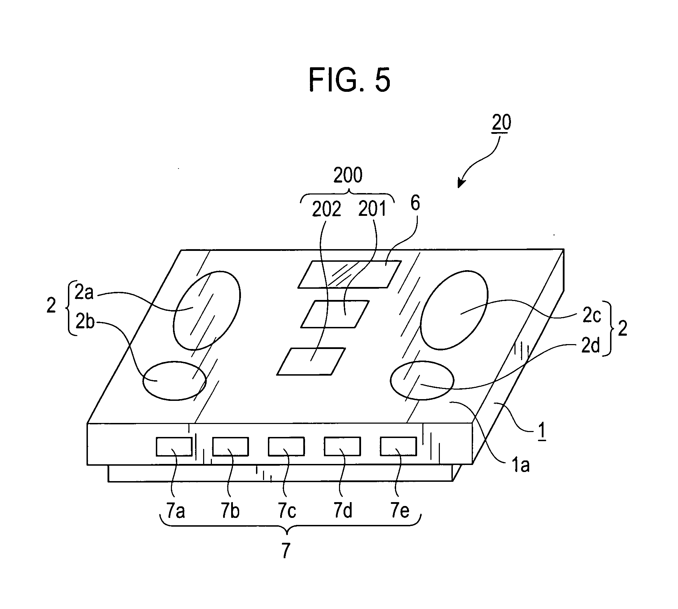

Fig. 5 is an external perspective view of a body composition data acquiring apparatus 20 as a second embodiment of the present invention. The body composition data acquiring apparatus 20 has the same constitution as that of the body composition data acquiring apparatus 10 (first embodiment) except that the apparatus 20 uses a length measuring unit 200 in place of the length measuring unit 100 used in the body composition data acquiring apparatus 10. Therefore, the same reference numbers are allocated to the same constituents as those found in the apparatus 10, and detailed descriptions of the constituents are omitted.

The length measuring unit 200 in this body composition data acquiring apparatus 20 comprises an ultrasonic wave transmitter 201 which is provided on the top surface 1a of the main unit 1 such that it transmits an ultrasonic wave upward and an ultrasonic wave receiver 202 which is also provided on the top surface 1a of the main unit 1 such that it receives an ultrasonic wave from above. These ultrasonic wave transmitter 201 and ultrasonic wave receiver 202 are situated between the electrodes 2a and 2b for the left foot and the electrodes 2c and 2d for the right foot. When a subject stands upright on the top surface 1a of the main unit, an ultrasonic wave transmitted from the ultrasonic wave transmitter 201 is reflected at the crotch of the subject and received by the ultrasonic wave receiver 202. At that time, a time required from the start of transmission of the ultrasonic wave to the start of receipt of the ultrasonic wave is clocked by the control unit 8, and in the control unit 8, the distance from the top surface 1a of the main unit to the crotch of the subject is measured (calculated) based on the clocked time. That is, the length measuring unit 200 comprising these ultrasonic wave transmitter 201 and ultrasonic wave receiver 202 and the control unit 8 primarily constitute leg length measuring means.

The measurement of the length from the crotch to the bottoms of the feet by the length measuring unit 200 and the control unit 8 is carried out automatically in STEP S7 of Fig. 3. To be more specific, after a message urging a subject to stand on the top surface 1a of the main unit and stay still for a given time (e.g., 15 seconds) is displayed on the display unit 6b in STEP S5, a body weight is measured, and a bioelectrical impedance is measured (STEP S6), and then transmission of ultrasonic wave is started and the length from the crotch to the bottoms of the feet is measured (STEP S7). In this case, such a message as displayed on the display unit 6b in STEP S7 of the body composition data acquiring apparatus 10 (first embodiment) is not displayed.

Embodiment 3

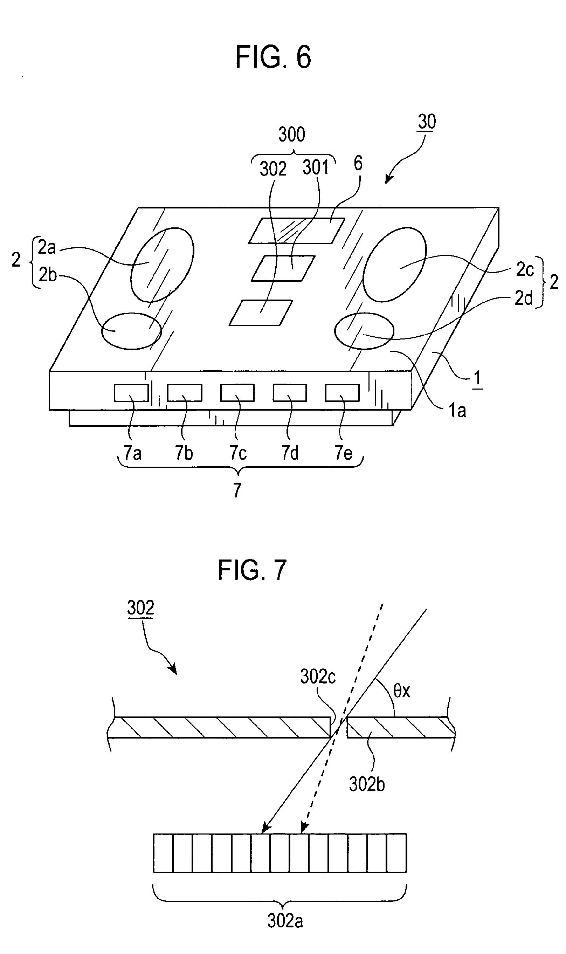

Fig. 6 is an external perspective view of a body composition data acquiring apparatus 30 as a third embodiment of the present invention. The body composition data acquiring apparatus 30 has the same constitution as that of the body composition data acquiring apparatus 10 (first embodiment) except that the apparatus 30 uses a length measuring unit 300 in place of the length measuring unit 100 used in the body composition data acquiring apparatus 10. Therefore, the same reference numbers are allocated to the same constituents as those found in the apparatus 10, and detailed descriptions of the constituents are omitted.

The length measuring unit 300 in this body composition data acquiring apparatus 30 comprises a light emitter 301 which is provided on the top surface 1a of the main unit 1 such that it emits light such as infrared light upward and a light receiver 302 which is also provided on the top surface 1a of the main unit 1 such that it receives light from above. These light emitter 301 and light receiver 302 are situated between the electrodes 2a and 2b for the left foot and the electrodes 2c and 2d for the right foot. When a subject stands upright on the top surface 1a of the main unit, light emitted from the light emitter 301 is reflected at the crotch of the subject and received by the light receiver 302.

In this case, the distance (L) between the light emitter 301 and the light receiver 302 and the light emitting angle (θ) in the light emitter 301 are fixed and the light receiving angle (θx) in the light receiver 302 is detected, whereby the distance (D) from the top surface 1a of the main unit to the crotch is measured (calculated) from these distance (L), light emitting angle (θ) and light receiving angle (θx) based on the following formula (1). That is, the length measuring unit 300 which comprises these light emitter 301 and light receiver 302 and the control unit 8 primarily constitute leg length measuring means.

$$D = L \times (\sin\theta \times \sin\theta x)/\sin(\theta \times \theta x) \qquad (1)$$

The light receiver 302 has such a constitution as shown in Fig. 7 so as to detect the light receiving angle (θx). To be more specific, the light receiver 302 comprises a light receiving element group 302a which comprises a plurality of light receiving elements arranged side by side and a plate 302b which has a slit 302c and covers the light receiving element group 302a. Light reflected at the crotch of a subject passes through the slit 302c and is detected by the light receiving element group 302a. At that time, as indicated by a solid line and a broken line in Fig. 7, the angle (i.e., light receiving angle θx) at which the light has passed through the slit 302c is specified by determining which light receiving element in the light receiving element group 302a has detected the light.

In the present embodiment, the distance (L) between the light emitter 301 and the light receiver 302 and the light emitting angle (θ) in the light emitter 301 are fixed. However, a mechanism for making them variable may be added. By making the distance (L) and/or the emitting angle (θ) variable, the measurable range of the distance (D) from the top surface 1a of the main unit to the crotch can be widened.

The measurement of the length from the crotch to the bottoms of the feet by the length measuring unit 300 and the control unit 8 is carried out automatically in STEP S7 of Fig. 3. To be more specific, after a message urging a subject to stand on the top surface 1a of the main unit and stay still for a given time (e.g., 15 seconds) is displayed on the display unit 6b in STEP S5, a body weight is measured, and a bioelectrical impedance is measured (STEP S6) , and then light is emitted from the light emitter 301 and the length from the crotch to the bottoms of the feet is measured (STEP S7) . In this case, such a message as displayed on the display unit 6b in STEP S7 of the body composition data acquiring apparatus 10 (first embodiment) is not displayed.

Embodiment 4

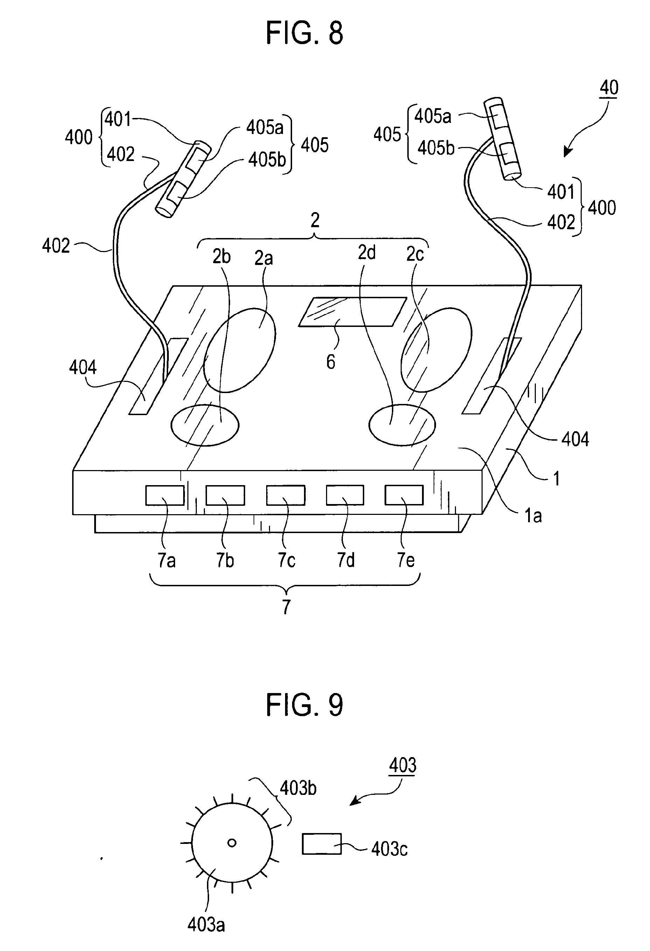

Fig. 8 is an external perspective view of a body composition data acquiring apparatus 40 as a fourth embodiment of the present invention. The body composition data acquiring apparatus 40 has the same constitution as that of the body composition data acquiring apparatus 10 (first embodiment) except that the apparatus 40 uses length measuring units 400 in place of the length measuring unit 100 used in the body composition data acquiring apparatus 10. Therefore, the same reference numbers are allocated to the same constituents as those found in the apparatus 10, and detailed descriptions of the constituents are omitted.

The length measuring unit 400 in this body composition data acquiring apparatus 40 comprises a flexible member 402 which can be pulled out of the main unit 1 and a pulled length sensor 403 (refer to Fig. 9) which detects the pulled length of the flexible member 402. The flexible member 402 has a grip 401 at one end. In the present embodiment, these grip 401, flexible member 402 and pulled length sensor 403 are provided for each of the left and right hands. Further, the pulled length sensor 403 comprises, as shown in Fig. 9, a reel drum 403a for reeling the flexible member 402, a plurality of teeth 403b which are formed on the outer perimeter in the axial direction of the reel drum 403a and a pickup sensor 403c which is positioned facing the teeth 403b. The teeth 403b are formed at intervals of, for example, 1°, except for one spot on the perimeter of the drum 403a.

Meanwhile, on the top surface 1a of the main unit 1, recesses 404 for storing the grips 401 and the flexible members 402 for the left and right hands are formed at the left side of the electrodes 2a and 2b for the left foot and at the right side of the electrodes 2c and 2d for the right foot. When not in use, the grips 401 and the flexible members 402 are stored in the recesses 404, and the flexible members 402 are wound around the reel drums 403a of the pulled length sensors. Further, in this state, the spot on the reel drum 403a where the tooth 403b is missing is adjusted to the position right in front of the pickup sensor 403c.

When a subject holds the grips 401 by both hands and pulls the flexible members 402 out of the recesses 404, the reel drums 403a rotate, and the teeth 403b pass in front of the pickup sensors 403c. Thus, in the control unit 8, the pulled length of the flexible member 402 is measured (calculated) from the numbers of the teeth and tooth-missing spots detected by the pickup sensor 403c. Therefore, when a subject holds the grips 401 by both hands and stands upright on the top surface 1a of the main unit with the grips on both sides of the waist, the positions of both hands are nearly equivalent to the height of the crotch, and the pulled length of the flexible member 402 at that time is measured as the length from the crotch to the bottom of the feet of the subject. That is, the lengthmeasuring unit 400 which comprises these grip 401, flexible member 402 and pulled length sensor 403 and the control unit 8 primarily constitute leg length measuring means.

Further, in the present embodiment, on each of the grips 401, a bioelectrical impedance measuring electrode group 405 which comprises an electrode 405a for supplying an alternating current to the palm and an electrode 405b for measuring the voltage of the palm is provided. Further, in the flexible member 402, a lead wire for supplying a current to the electrode 405a and a lead wire for measuring a voltage by the electrode 405b are incorporated. Via these lead wires, the electrodes 405a are connected to the current supplying unit 3 of the main unit 1, and the electrodes 405b are connected to the voltage measuring unit 4 of the main unit 1 (refer to broken lines in Fig. 2). That is, the body composition data acquiring apparatus 40 of the present embodiment is constituted as a so-called 8-electrode-type body composition data acquiring apparatus which can not only measure a bioelectrical impedance between both feet by use of the electrode group 2 but also measure a bioelectrical impedance between both hands by use of the electrode group 405 and a bioelectrical impedance between the hand and the foot by use of the electrode group 2 and the electrode group 405.

The measurement of the length from the crotch to the bottoms of the feet by the length measuring unit 400 and the control unit 8 is carried out automatically in STEP S7 of Fig. 3. To be more specific, in STEP S5, after a message urging a subj ect to hold the grips 401 by both hands, pull out the flexible members 402, stand upright on the top surface 1a of the main unit with the hands on both sides of the waist and the bottoms of the feet in contact with the electrode group 2 and stay still for a given time (e.g., 20 seconds) is displayed on the display unit 6b, a body weight is measured. In subsequent STEP S6, a bioelectrical impedance between both feet, a bioelectrical impedance between both hands and a bioelectrical impedance between the hand and the foot are measured. Subsequently, in STEP S7, the pulled length of the flexible member 402 is measured by the pulled length sensor 403 as the length from the crotch to the bottoms of the feet. In case the pulled lengths of the left and right flexible members 402 are different, their average is measured as the length from the crotch to the bottoms of the feet.

The body composition data acquiring apparatus 40 according to the present embodiment is constituted as an 8-electrode-type body composition data acquiring apparatus by providing the electrode group 405 on the left and right grips 401. However, for measurement of only the length of the leg, the apparatus 40 may be constituted as a 4-electrode-type body composition data acquiring apparatus by omitting these electrode groups 405. Further, in the present embodiment, although the length measuring unit 400 which comprises the grip 401, the flexible member 402 and the pulled length sensor 403 is provided for each of the left and right hands, it may be provided for only either of the hands.

**Claims**

1. A body composition data acquiring apparatus having a main unit comprising:

    bioelectrical impedance measuring means,
    body weight measuring means,
    leg length measuring means, and
    body composition data calculating means

   wherein

    the bioelectrical impedance measuring means measures a
    bioelectrical impedance between both feet by use of a group of electrodes which make contact with the bottoms of the feet of a subject,
    the body weight measuring means measures the body weight of the subject,
    the leg length measuring means measures the leg length of the subject, and
    the body composition data calculating means calculates the body composition data of the subject based on at least the
    bioelectrical impedance, body weight and leg length measured by these measuring means.

2. The apparatus of claim 1, wherein the leg length measuring means measures the length from the crotch to the bottoms of the feet of the subject.

3. The apparatus of claim 1, wherein the leg length measuring means measures the length from the knees to the bottoms of the feet of the subject.

4. The apparatus of claims 1 to 3, wherein the leg length measuring means comprises:

    a pole which is positioned nearly perpendicularly to the main unit,
    a cursor which is slidably attached to the pole, and
    a position sensor which detects the position of the cursor.

5. The apparatus of claim 1 or 2, wherein the leg length. measuring means comprises:

    an ultrasonic wave transmitter which is provided on the top surface of the main unit and transmits an ultrasonic wave upward, and
    an ultrasonic wave receiver which is provided on the top surface
    of the main unit and receives an ultrasonic wave from above.

6. The apparatus of claim 1 or 2, wherein the leg length measuring means comprises:

a light emitter which is provided on the top surface of the main unit and emits light upward, and
a light receiver which is provided on the top surface of the main unit and receives light from above.

7. The apparatus of claim 1 or 2, wherein the leg length measuring means comprises:

a flexible member which can be pulled out of the main unit and has a grip at one end, and
a pulled length sensor which detects the pulled length of the flexible member.

8. The apparatus of claim 7, wherein the grip of the flexible member has a group of electrodes for measuring a bioelectrical impedance.

# FIG. 1

# FIG. 2

| | | | | | | | |
|---|---|---|---|---|---|---|---|
| 2a | 2c | (405a) | (405a) | 2b | 2d | (405b) | (405b) |

3 — CURRENT SUPPLYING UNIT

VOLTAGE MEASURING UNIT — 4

LOAD SENSOR UNIT — 5

8

CONTROL UNIT

LENGTH MEASURING UNIT

100,200,300,400

6c — INPUT/OUTPUT CONTROL UNIT

STORAGE UNIT — 9

INPUT UNIT

DISPLAY UNIT

6a

6b

EP 1 514 513 A1

# FIG. 3

```
        ┌──────────────────┐
        │     POWER ON     │
        └──────────────────┘
                 │
                 ▼
S1 ──┌──────────────────┐
     │  INITIALIZATION  │
     └──────────────────┘
                 │
                 ▼
S2 ──◇ PERSONAL DATA ────── YES ──┐
     ◇ ALREADY STORED ?          │
                 │ NO             │
                 ▼                │
S3 ──┌──────────────────┐         │
     │   SELECT GENDER  │         │
     └──────────────────┘         │
                 │                │
                 ▼                │
S4 ──┌──────────────────┐         │
     │     ENTER AGE    │         │
     └──────────────────┘         │
                 │                │
                 ▼◄───────────────┘
S5 ──┌──────────────────────┐
     │ MEASURE BODY WEIGHT  │
     └──────────────────────┘
                 │
                 ▼
S6 ──┌──────────────────────┐
     │  MEASURE IMPEDANCE   │
     └──────────────────────┘
                 │
                 ▼
S7 ──┌──────────────────────┐
     │  MEASURE LEG LENGTH  │
     └──────────────────────┘
                 │
                 ▼
S8 ──┌──────────────────────┐
     │   CALCULATE BODY     │
     │  COMPOSITION DATA    │
     └──────────────────────┘
                 │
                 ▼
S9 ──┌──────────────────────┐
     │    DISPLAY BODY      │
     │  COMPOSITION DATA    │
     └──────────────────────┘
                 │
                 ▼
        ┌──────────────────┐
        │     POWER OFF    │
        └──────────────────┘
```

## FIG. 4A

SELECT YOUR GENDER.

MALE  FEMALE
▲

## FIG. 4B

ENTER YOUR AGE.

28 YEARS OLD

NEXT  RETURN
▲

## FIG. 4C

MEASURING BODY WEIGHT
AND IMPEDANCE ...

PLEASE STAY STILL.

PROGRESS ON
MEASUREMENT
■■■■■

## FIG. 4D

MEASURING LENGTH FROM
CROTCH TO BOTTOMS OF FEET ...

PLEASE STAY STILL.

PROGRESS ON
MEASUREMENT
■■■□□

## FIG. 4E

| BODY WEIGHT | 60kg |
|---|---|
| BODY FAT PERCENTAGE | 20% |
| MUSCLE MASS | 44kg |
| BASAL METABOLIC RATE | 1750kcal |
| VISCERAL FAT AREA | $70cm^2$ |
| BCM | 40kg |
| BONE MASS | 4kg |

# FIG. 5

20

200

202  201  6

2 { 2a
    2b

2c } 2
2d

1

1a

7a  7b  7c  7d  7e

7

# FIG. 6

# FIG. 7

# FIG. 8

# FIG. 9

**European Patent Office**

**EUROPEAN SEARCH REPORT**

Application Number

EP 04 02 0164

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 1999, no. 10, 31 August 1999 (1999-08-31) & JP 11 128197 A (A & D CO LTD), 18 May 1999 (1999-05-18) * abstract * * paragraphs [0001], [0002], [0017], [0020], [0042] * * figure 1 * | 1-3,5 | A61B5/053 |
| Y | | 4,6-8 | |
| A | PATENT ABSTRACTS OF JAPAN vol. 1999, no. 11, 30 September 1999 (1999-09-30) & JP 11 155829 A (A & D CO LTD), 15 June 1999 (1999-06-15) * abstract * * figures 3,5 * | 1 | |
| A,D | PATENT ABSTRACTS OF JAPAN vol. 2000, no. 19, 5 June 2001 (2001-06-05) & JP 2001 029321 A (MATSUSHITA ELECTRIC IND CO LTD), 6 February 2001 (2001-02-06) * abstract * * paragraph [0012] * * figure 5 * | 2 | TECHNICAL FIELDS SEARCHED (Int.Cl.7)<br><br>A61B |
| Y | EP 1 201 187 A (TANITA SEISAKUSHO KK) 2 May 2002 (2002-05-02) * column 10, line 43 - column 11, line 10 * * figure 13 * | 4 | |

-/--

The present search report has been drawn up for all claims

| Place of search | Date of completion of the search | Examiner |
|---|---|---|
| The Hague | 13 October 2004 | Quentric, F-X |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## EUROPEAN SEARCH REPORT

European Patent Office

Application Number

EP 04 02 0164

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | PATENT ABSTRACTS OF JAPAN<br>vol. 2000, no. 08,<br>6 October 2000 (2000-10-06)<br>& JP 2000 139869 A (OMRON CORP),<br>23 May 2000 (2000-05-23)<br>* abstract *<br>* paragraphs [0156], [0172], [0173] *<br>* figure 33 *<br>----- | 6-8 | |

TECHNICAL FIELDS
SEARCHED      (Int.Cl.7)

The present search report has been drawn up for all claims

| Place of search | Date of completion of the search | Examiner |
|---|---|---|
| The Hague | 13 October 2004 | Quentric, F-X |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 04 02 0164

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2004

| Patent document cited in search report | | Publication date | Patent family member(s) | | Publication date |
|---|---|---|---|---|---|
| JP 11128197 | A | 18-05-1999 | NONE | | |
| JP 11155829 | A | 15-06-1999 | NONE | | |
| JP 2001029321 | A | 06-02-2001 | JP | 3508633 B2 | 22-03-2004 |
| EP 1201187 | A | 02-05-2002 | JP | 2002125947 A | 08-05-2002 |
| | | | EP | 1201187 A1 | 02-05-2002 |
| | | | US | 2002049546 A1 | 25-04-2002 |
| JP 2000139869 | A | 23-05-2000 | NONE | | |

EPO FORM P0459

For more details about this annex : see Official Journal of the European Patent Office, No. 12/82